# EUROPÄISCHE PATENTANMELDUNG

(11) **EP 2 886 154 A1**
(43) Veröffentlichungstag der Anmeldung: **24.06.2015**
(21) Anmeldenummer: 14003940.5
(22) Anmeldetag: 24.11.2014
(51) Int. Cl.: A61N 1/04, A61N 1/36, A61N 1/08

(54) **Vorrichtung zur Aufbringung eines transkutanen elektrischen Stimulationsreizes**

(30) Priorität: 17.12.2013 DE 102013021175
(71) Anmelder: cerboMed GmbH, 91052 Erlangen (DE)
(72) Erfinder: Hartlep, Andreas, 83607 Holzkirchen (DE); Frenkel, Wolf Gerhard, 72514 Inzigkofen-Engelswies (DE); Zschaeck, Thomas, 90427 Nürnberg (DE); Hyca, Martin, 91054 Erlangen (DE)
(74) Vertreter: Gosdin, Michael

(57) **Zusammenfassung**

Die Erfindung betrifft eine Vorrichtung (1) zur Aufbringung eines transkutanen elektrischen Stimulationsreizes auf die Oberfläche eines Abschnittes des menschlichen Ohrs (2), die eine Anzahl Elektroden (3, 4) aufweist, die an oder in einem Elektrodenträger (5) angeordnet sind, wobei die Vorrichtung (1) eine Steuerungseinrichtung (6) umfasst, die die Herstellung einer Potentialdifferenz zwischen den Elektroden (3, 4) steuert oder regelt, wobei die Vorrichtung (1) Signalmittel (7) umfasst, die den zwischen den Elektroden (3, 4) herrschenden Widerstand bei anliegender Potentialdifferenz bei ans oder ins Ohr (2) angesetzter Vorrichtung (1) anzeigt oder angibt, wobei die Signalmittel (7) mindestens ein Signalelement (7', 7", 7"') aufweisen, das in Abhängigkeit des gemessenen Widerstands angesteuert wird. Um die Stimulationsqualität der transkutanen Stimulation durch den Benutzer der Vorrichtung optimal einstellen zu können, sieht die Erfindung vor, dass die Signalmittel (7) eine Mehrzahl an Signalelementen (7', 7", 7'") aufweisen, wobei in Abhängigkeit von vordefinierten Bandbreiten des Widerstands unterschiedliche Signalelemente (7', 7", 7'") angesteuert werden.

## Beschreibung

Die Erfindung betrifft eine Vorrichtung zur Aufbringung eines transkutanen elektrischen Stimulationsreizes auf die Oberfläche eines Abschnittes des menschlichen Ohrs, die eine Anzahl Elektroden aufweist, die an oder in einem Elektrodenträger angeordnet sind, wobei die Vorrichtung eine Steuerungseinrichtung umfasst, die die Herstellung einer Potentialdifferenz zwischen den Elektroden steuert oder regelt, wobei die Vorrichtung Signalmittel umfasst, die den zwischen den Elektroden herrschenden Widerstand bei anliegender Potentialdifferenz bei ans oder ins Ohr angesetzter Vorrichtung anzeigt oder angibt, wobei die Signalmittel mindestens ein Signalelement aufweisen, das in Abhängigkeit des gemessenen Widerstands angesteuert wird.

Es ist generell bekannt, durch invasive und non-invasive Reizung der Nerven Einfluss auf deren neurophysiologische und neuroelektrische Qualität und damit auf die Funktion der stimulierten Nerven zu nehmen. Hierdurch können verschiedene Krankheitszustände behandelt werden. Es existieren zahlreiche Vorrichtungen sowohl zur invasiven als auch zur non-invasiven Stimulation. Die vorliegende Erfindung stellt auf die Methode der transkutanen elektrischen Nervenstimulation ab. Bei diesem Verfahren werden Impulsströme verschiedener Stromformen, Amplituden, Impulsdauern und Frequenzen durch die Haut hindurch an verschiedenen Nerven appliziert und verändern deren Statusparameter in vorteilhafter Weise.

Eine Vorrichtung der eingangs genannten Art offenbaren **die** US 5 514 175 A und die US 2009/0082831 A1. Eine ähnliche Vorrichtung ist aus der DE 10 2010 054 165 B3 bekannt. Hier ist eine Vorrichtung zur transkutanen Stimulation des Vagusnervs des menschlichen Körpers beschrieben, deren Elektrodenkopf mit zwei Elektroden im Bereich der Cymba conchae angeordnet wird; eine solche Positionierung der Elektroden hat sich als vorteilhaft erwiesen. Der Bereich der Cymba conchae ist dabei der Bereich der Concha des Ohres, der oberhalb des Crus helicis liegt; er wird auch als Hemiconcha superior bezeichnet. Unterhalb des Crus helicis nach unten erstreckt sich dann der Bereich des Cavum conchae. Schließlich zeigt auch die DE 10 2011 018 228 A1 eine ähnliche Lösung.

Es hat sich dabei als schwierig erwiesen, den Elektrodenkopf mit den Elektroden so im Ohr anzuordnen, dass eine optimale Kontaktqualität für die transkutane Stimulation gegeben ist. Also optimal ist dabei nicht nur eine solche Kontaktierung der Elektroden anzusprechen, die keinen zu hohen Widerstand zwischen den Elektroden hervorruft; genauso ist auch ein zu kleiner Widerstand zwischen den Elektroden nachteilig und somit nicht optimal.

Der vorliegenden Erfindung liegt daher die **Aufgabe** zugrunde, eine Vorrichtung der genannten Art bereitzustellen, mit der es für den Arzt oder für den Patienten selber in einfacher Weise möglich ist, die Stimulationsvorrichtung so im Ohr zu positionieren, dass eine bestmögliche Kontaktqualität für die Stimulation erreicht werden kann.

Die **Lösung** dieser Aufgabe durch die Erfindung ist dadurch gekennzeichnet, dass die Signalmittel eine Mehrzahl an Signalelementen aufweisen, wobei in Abhängigkeit von vordefinierten Bandbreiten des Widerstands unterschiedliche Signalelemente angesteuert werden.

Die Signalmittel können dabei mit der Vorrichtung fest verbunden sein; es ist aber auch möglich, dass die Signalmittel von der Vorrichtung trennbar oder getrennt sind und über die Steuerungseinrichtung und die Signalmittel über eine kabelgebundene oder drahtlose Verbindung miteinander kommunizieren können. In letzterem Falle können die Signalmittel von der Vorrichtung getrennt und bei der Suche einer optimalen Position für die transkutane Stimulation besser beobachtet werden.

Ferner ist es für eine gute Beobachtung der Signalmittel während der Platzierung der Elektroden auf der Hautoberfläche vorteilhaft, wenn gemäß einer Fortbildung der Elektrodenträger mit den Elektroden einerseits (also die Otoplastik) und die Steuerungseinrichtung andererseits (also der eigentliche Stimulator) als separate Bauteile ausgebildet sind, die miteinander in Verbindung stehen, wobei die Signalmittel mit der Steuerungseinrichtung fest verbunden sind.

Die Signalelemente können dabei Leuchtdioden sein, wobei die Leuchtdioden vorzugsweise Licht unterschiedlicher Farbe emittieren. Es können dabei drei Leuchtdioden vorhanden sein. Die drei Leuchtdioden können grünes, gelbes und rotes Licht emittieren, und als "Ampel" (s. unten) fungieren. Auch eine Ausstattung mit fünf verschieden farbigen Leuchtdioden hat sich bewährt, um in einfacherer Weise die Richtung der Optimierung erkennen zu können.

Die Signalmittel können auch ein Signalelement zur Anzeige einer Ziffer aufweisen. Hiermit kann eine Ziffer zwischen "0" und "9" angezeigt werden, um die Kontaktqualität beurteilen zu können.

Das mindestens eine Signalelement kann auch nach einer anderen Ausgestaltung ein akustischer Geber sein; hierbei ist insbesondere an ein Piezokristall gedacht. Die Steuerungseinrichtung kann dabei ausgebildet sein, in Abhängigkeit des zwischen den Elektroden herrschenden Widerstands den akustischen Geber zu veranlassen, unterschiedliche Frequenzen abzugeben.

Das mindestens eine Signalelement kann weiterhin nach einer anderen Ausführungsform ein Geber sein, der ein vom Benutzer der Vorrichtung spürbares Signal abgibt. Hierbei ist insbesondere an einen Geber gedacht, der Vibrationen aussendet. Demgemäß wären dann vom Benutzer der Vorrichtung taktile Reize wahrnehmbar, die Auskunft über die Kontaktqualität geben (derartige Lösungen sind als Vibrationsmittel bekannt, wie sie in Mobiltelefonen eingesetzt werden).

Als weitere alternative Ausführungsform ist es möglich, dass das besagte Signalelement als Bildschirm ausgebildet ist, auf dem im Klartext Hinweise zur Veränderung der Lage der Elektroden auf der Haut gegeben werden.

Erfindungsgemäß geht es also generell um die Optimierung der Stimulationsqualität, was dadurch erreicht werden soll, dass dem Benutzer bzw. Patient der Vorrichtung eine Rückmeldung (Feedback) von der Vorrichtung über die Kontaktqualität gegeben wird. Dabei werden dem Benutzer gemäß einer bevorzugten Ausführungsform der Erfindung auch Hinweise gegeben, wie er gegebenenfalls die Position der Elektroden auf der Hautoberfläche ändern sollte, um zu einem besseren Ergebnis zu kommen.

Die vorgeschlagene Vorrichtung kann dabei gemäß einer bevorzugten Ausführungsform nach Art einer "Ampel" arbeiten, wobei in diesem Falle beispielsweise drei Leuchtdioden vorhanden sind, die den Status der Kontaktqualität signalisieren. Dabei können drei Farben vorgesehen werden ("grün" - "gelb" - "rot"), wobei zwecks Anzeige von Zwischenzuständen auch zwei der Leuchtdioden gleichzeitig leuchten können (nämlich "grün und gelb" oder "gelb und rot"). Dies liefert dem Benutzer einen Hinweis, ob eine Re-Positionierung der Elektroden auf der Hautoberfläche zu einer verbesserten oder verschlechterten Kontaktqualität führt.

Zeigt die "Ampel" zunächst "rot" an, verschiebt der Anwender - durchaus auch im Wege des "Trial and Error" den Elektrodenkopf auf der Hautoberfläche, bis "gelb" (mit) aufleuchtet. Der Benutzer kann dann die vorgenommene Positionsänderung weiter extrapolieren, um zu sehen, ob nach dem (vollständigen) Erlöschen von "rot" dann "grün" (mit) aufleuchtet. Leuchtet "grün" auf, kann der Benutzer sicher sein, dass für die sich anschließende Stimulation eine optimale Basis vorliegt.

Generell ist es auch möglich, dass als Signalmittel nicht nur mehrere Leuchtmittel (Leuchtdioden) mit verschiedenen Farben verwendet werden; es kann auch nur eine Leuchtdiode verwendet werden, die in unterschiedlichen Farben leuchten kann. Ferner kann auch vorgesehen werden, dass die Intensität des Leuchtens variiert wird, um ein entsprechendes Signal zu liefern.

Die "Ampel" kann auch so realisiert werden, dass diese auf einem (Farb)Display dargestellt wird. Alternativ kann ferner - dann beispielsweise unter Nutzung einer monochromen Anzeige - statt einer "Ampel" ein Balken dargestellt werden, der abhängig vom gemessenen Widerstand länger oder kürzer ist.

Betreffend das Auffinden einer verbesserten Position der Elektroden auf der Hautoberfläche können vom Anwender unter anderem folgende Maßnahmen ergriffen werden:

Die Positionsänderung des Elektrodenpaars relativ zur Haut - insbesondere im Bereich der Cymba conchae - kann eine Translationsbewegung des Elektrodenkopfs umfassen; diese Bewegung kann generell in allen Richtungen auf der Hautoberfläche und parallel zu dieser vorgesehen werden. Der Stimulationsort kann dann auch durch zirkuläres Verschwenken der Elektroden bzw. des Elektrodenträgers an einem Ort verändert werden. Es kann auch eine Verschwenkung der durch die Elektroden gebildeten Ebene der Elektroden relativ zur Hautebene vorgenommen werden, um ein höheres Maß an Parallelität zur Hautoberfläche zu erreichen. Es kann auch eine Veränderung - meist in Form einer Erhöhung - des Anpressdrucks der Elektroden auf der Haut ins Auge gefasst werden.

Dies kann auch von flankierenden Maßnahmen begleitet sein: So kann eine Entfettung der Hautoberfläche vorgenommen werden. Andererseits kommt auch die Applikation eines Kontaktmediums in angemessen gewählter Weise in Frage (es ist in diesem Falle eine hinreichende Menge Kontaktmedium zu wählen, um eine Kontaktverbesserung zu erreichen; andererseits ist aber auch zu viel Kontaktmedium zu vermeiden, um einen "Kurzschluss" zwischen den Elektroden über die Hautoberfläche zu verhindern, was den angestrebten therapeutischen Effekt untergraben würde).

Vorgesehen kann auch werden, dass die Elektroden mit strukturellen Mitteln zu Erhöhung bzw. zur Vergleichmäßigung der Kontaktbedingungen versehen werden (z. B. eine "Hülle" für den Elektrodenträger).

Die Anzeige mittels des erfindungsgemäß vorgesehenen Signalmittels bzw. den Signalelementen liefern dabei im Ergebnis einen Indikator hinsichtlich des elektrischen Widerstandes zwischen den Elektroden der Stimulationsvorrichtung (bzw. für dessen Kehrwert, d. h. für die elektrische Leitfähigkeit).

Es kann dabei auch eine Anzeige der Spannung erfolgen - was über das Ohmsche Gesetz wiederum auf die Größe des Widerstandes zwischen den Elektroden hinausläuft -, die zur Erzeugung eines definierten Stroms (d. h. eines Prüfstroms) zwischen den Elektroden angelegt werden muss.

So kann beispielsweise ein Prüfstrom von 100 µA zugrunde gelegt werden und mittels der Signalelemente die Spannung angezeigt werden, die zur Erzielung besagten Prüfstroms erforderlich ist. Über das Ohmsche Gesetz (U = R x I) kann dann auch eine Aussage über den vorhandenen Widerstand bzw. die Impedanz gemacht werden.

Ist eine zu hohe Spannung erforderlich (über einen vorgegebenen Grenzwert), liefern die Signalelemente die Anzeige "kein Kontakt" - "rote Ampel" (d. h. zu hoher Widerstand). Allerdings würde das Gerät auch dann die "rote Ampel" anzeigen, wenn der Strom und damit der Widerstand zu niedrig ist, d. h. wenn "Kurzschluss" vorliegt.

Für die "Ampel" kann dann beispielsweise bei dem genannten Prüfstrom von 100 µA ein Wertebereich zugrunde gelegt werden:
- Grün: bis 40 V
- Gelb: 40 bis 60 V
- Rot: über 60 V

Wie erläutert, muss der optimale Stimulationsbereich allerdings nicht nur nach oben (in Richtung hoher Spannungen), sondern auch nach unten abgegrenzt werden, wozu auf die obigen Ausführungen zum "Kurzschluss" verwiesen wird. Ist die Kontaktqualität nämlich zu gut, womöglich sogar schon vor dem Kontakt zum Ohr (beispielsweise, wenn zu viel Kontaktgel aufgetragen wurde, das eine elektrisch leitende Verbindung zwischen den Elektroden schon vor dem Einsetzen in das Ohr herstellt), sollte der Benutzer der Vorrichtung ebenfalls Hinweise auf die Optimierung bekommen, analog zur zu hohen Spannung, dann aber eben auch bei zu niedriger Spannung.

Es lässt sich also folgendes sagen: Die Herstellung günstiger Verhältnisse für eine effiziente transkutane Stimulation kann zur einen Seite hin eindeutig definiert werden. Hierfür lässt sich eine definierte Spannung angeben, die für das Erzielen einer Prüfstromstärke angelegt werden muss. In diesem Falle liegt bis zu einem zulässigen Grenzwert der Widerstand, ablesbar an der erforderlichen Spannung zur Erreichung des Sollstroms, im therapeutisch wirksamen Bereich.

Bei Vorliegen von ungünstigen Verhältnissen für eine effiziente transkutane Stimulation müssen zwei Fälle unterschieden werden.

Zum einen kann der Widerstand (entsprechend der erforderlichen Spannung zur Erreichung eines Sollstroms) zu hoch sein, weil die Kontaktqualität zwischen Haut und Elektrode nicht ausreicht.

Zum anderen kann aber auch der Widerstand zu gering sein; es besteht dann ein Kurzschluss über die Hautoberfläche von der Stimulations- zur Referenzelektrode, ohne dass die Haut und damit die rezeptiven Felder der Nerven vom Strom durchflossen werden.

Demgemäß kann in Fortbildung des vorgeschlagenen Konzepts vorgesehen sein, dass die "Ampel" nicht nur drei Farben aufweist ("grün - gelb - rot"), womit generell nur eine zu schlechte und eine zu gute Leitfähigkeit abgebildet werden können. Zusätzlich kann vorgesehen werden, dass zwei weitere Leuchtdioden zum Einsatz kommen, die die Ampelkonstellation "rot" differenzieren nach "zu hohem Widerstand" und "zu niedrigem Widerstand" im Sinne der obigen Ausführungen. In diesem Falle wäre die "Ampel" mit fünf Lichtern ausgestattet.

In diesem Zusammenhang sei folgendes erwähnt: Die gemessenen Widerstandswerte können abhängig von den konkreten Umständen und vom individuellen Benutzer Streuungen aufweisen, so dass es mitunter schwierig ist, im Vorfeld, also vor einer konkreten Applikation, Wertebereiche für den Widerstand bzw. die Kontaktspannung festzulegen, die per Anzeigeelemente angezeigt werden. Demgemäß sieht eine Weiterbildung vor, dass die Anzeigebereiche für die gemessenen Werte (d. h. im Falle der "Ampel": grün, gelb oder rot) individuell festgelegt bzw. zumindest angepasst, d. h. eingestellt werden. Das System wäre also insoweit lernfähig bzw. es kann sich im Sinne einer selbstlernenden Software selbst an konkrete Umstände anpassen. Hierbei kann also vorgesehen werden, dass zum Betrieb der Stimulationsvorrichtung eine Software eingesetzt wird, die "selbstlernend" ist und entsprechende Anpassung automatisch vornimmt.

Hierbei kann es sich natürlich anbieten, statt einer dreigliedrigen Anzeige (wie im Falle der klassischen "Ampel") eine feinere - gegebenenfalls sehr viel feinere - Untergliederung vorzunehmen. Dies kann bis dahin entwickelt werden, dass ein Wert zwischen "0 %" und "100 %" angezeigt wird, der präzisere Rückschlüsse auf die Kontaktqualität zulässt.

Da sich diese Anzeige in Echtzeit ändert, würde dem Benutzer der Vorrichtung bzw. dem Arzt ständig ein Hinweis gegeben, ob er auf dem richtigen Weg bei der Suche nach einer optimalen Positionierung der Elektroden ist.

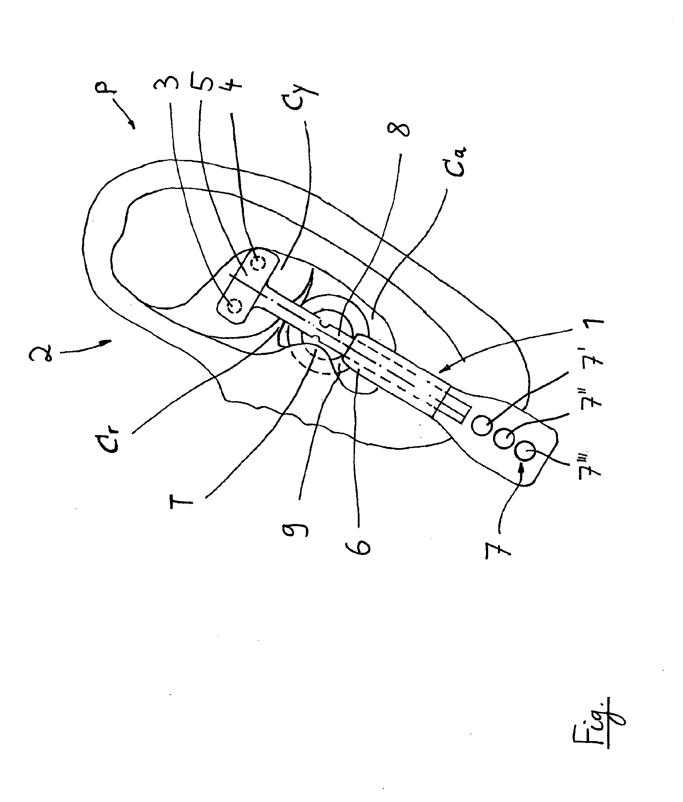

In der Zeichnung ist ein Ausführungsbeispiel der Erfindung dargestellt. Die einzige Figur zeigt die Ansicht einer Pinna (Ohrmuschel), in die eine Vorrichtung zur Aufbringung eines transkutanen Stimulationsreizes eingesetzt ist.

In der Figur ist eine Vorrichtung 1 zur transkutanen Stimulation eines Abschnitts des menschlichen Ohrs 2 skizziert. Die Vorrichtung 1 weist eine Haltestange 8 auf, die in einem Basisteil längsverschieblich ist, das auch eine Steuerungseinrichtung 6 umfasst. Am Basisteil ist ein Auflageteil 9 angeordnet. Die generelle Positionierung der Vorrichtung 1 im Ohr 2 ergibt sich aus der Markierung der wesentlichen Bereiche des Ohrs 2, nämlich der Pinna P mit dem Cavum conchae Ca, der Cymba conchae Cy, dem Tragus T und dem Crus helicis Cr.

Am einen Ende der Haltestange 8 ist ein Elektrodenträger 5 angeordnet, der zwei Elektroden 2 und 4 aufweist, zwischen denen zwecks transkutaner Stimulation eine Potentialdifferenz aufgebaut wird.

Insoweit entspricht die Vorrichtung 1 zunächst vorbekannten Lösungen, wobei insbesondere und ausdrücklich auf die DE 10 2010 054 165 B3 der Patentinhaberin Bezug genommen wird, wo eine solche Vorrichtung näher erläutert ist.

Die Vorrichtung 1 ist demnach ausgebildet, um im Bereich des Vagusnervs am Ohr 2 der die Vorrichtung benutzenden Person angebracht zu werden. Damit kann eine transkutane Stimulation des Vagusnervs vorgenommen werden.

Wesentlich ist, dass die Vorrichtung 1 vorliegend Signalmittel 7 umfasst, die den zwischen den Elektroden 3 und 4 herrschenden elektrischen Widerstand anzeigt bzw. angibt, wenn die Vorrichtung ans bzw. ins Ohr 2 angesetzt ist. Diese Signalmittel 7 weisen im Ausführungsbeispiel drei Signalelemente 7', 7" und 7'" auf, die in Abhängigkeit des gemessenen Widerstands angesteuert werden.

Die drei Signalelemente 7', 7", 7"' sind im Ausführungsbeispiel drei Leuchtdioden unterschiedlicher Farbe, nämlich grün (7'), gelb (7") und rot (7"').

Beim Bewegen des Elektrodenträgers 5 mit den Elektroden 3 und 4 verändert sich der gemessene elektrische Widerstand, so dass abhängig hiervon - im Sinne der obigen Erläuterungen - eine (bzw. auch gleichzeitig zwei) der Leuchtdioden angesteuert wird. Der Benutzer kann somit sofort sehen, ob für die Durchführung einer transkutanen Stimulation gute oder zumindest brauchbare Bedingungen vorliegen.

Bei "grün" sind die Kontaktverhältnisse gut, die Stimulation kann effizient erfolgen.

Bei "gelb" verschlechtern sich die Kontaktbedingungen, bei "rot" sind keine brauchbaren Kontaktbedingungen gegeben.

Wie oben erläutert, können beispielsweise zwei weitere Leuchtdioden (nicht dargestellt) vorgesehen werden, um anzuzeigen, dass im Falle unbrauchbarer Kontaktbedingungen (also bei "rot") ein zu hoher oder ein zu niedriger Widerstand vorliegt.

Bei zu hohem Widerstand kann mittels Kontaktgel Abhilfe geschaffen werden, bei zu niedrigem Widerstand muss gegebenenfalls eine Reinigung / Entfettung der Elektroden bzw. des Elektrodenträgers (von Kontaktgel) bzw. der Hautoberfläche erfolgen.

Vorstellbar ist in diesem Zusammenhang auch, dass es bei den drei genannten Leuchtdioden 7', 7" und 7'" bleibt und die Zusatzinformation über einen zu hohen Widerstand bzw. einen zu geringen Widerstand durch ein akustisches Signal ausgegeben wird. Demgemäß kann nach dem vorgeschlagenen Konzept auch vorgesehen sein, dass optische und akustische Signalelemente kombiniert werden. Durch Bewegen des Elektrodenträgers 5 mit den Elektroden 3 und 4 über die Hautoberfläche der Cymba conchae Cy kann so bei Beobachtung der Signalmittel 7 die optimale Position für die Stimulation aufgefunden werden.

### Bezugszeichenliste:

- 1: Vorrichtung zur transkutanen Stimulation
- 2: Ohr
- 3: Elektrode
- 4: Elektrode
- 5: Elektrodenträger
- 6: Steuerungseinrichtung
- 7: Signalmittel
- 7': Signalelement
- 7'': Signalelement
- 7''': Signalelement
- 8: Haltestange
- 9: Auflageteil

- Ca: Cavum conchae
- Cy: Cymba conchae
- T: Tragus
- Cr: Crus helicis
- P: Pinna

## Patentansprüche

1. Vorrichtung (1) zur Aufbringung eines transkutanen elektrischen Stimulationsreizes auf die Oberfläche eines Abschnittes des menschlichen Ohrs (2), die eine Anzahl Elektroden (3, 4) aufweist, die an oder in einem Elektrodenträger (5) angeordnet sind, wobei die Vorrichtung (1) eine Steuerungseinrichtung (6) umfasst, die die Herstellung einer Potentialdifferenz zwischen den Elektroden (3, 4) steuert oder regelt, wobei die Vorrichtung (1) Signalmittel (7) umfasst, die den zwischen den Elektroden (3, 4) herrschenden Widerstand bei anliegender Potentialdifferenz bei ans oder ins Ohr (2) angesetzter Vorrichtung (1) anzeigt oder angibt, wobei die Signalmittel (7) mindestens ein Signalelement (7', 7", 7'") aufweisen, das in Abhängigkeit des gemessenen Widerstands angesteuert wird,
**dadurch gekennzeichnet, dass**
die Signalmittel (7) eine Mehrzahl an Signalelementen (7', 7", 7"') aufweisen, wobei in Abhängigkeit von vordefinierten Bandbreiten des Widerstands unterschiedliche Signalelemente (7', 7", 7"') angesteuert werden.

2. Vorrichtung nach Anspruch 1, **dadurch gekennzeichnet, dass** die Signalmittel (7) mit der Vorrichtung (1) fest verbunden sind.

3. Vorrichtung nach Anspruch 1, **dadurch gekennzeichnet, dass** die Signalmittel (7) von der Vorrichtung (1) trennbar oder getrennt sind und über die Steuerungseinrichtung (6) und die Signalmittel (7) über eine kabelgebundene oder drahtlose Verbindung miteinander kommunizieren können.

4. Vorrichtung nach einem der Ansprüche 1 bis 3, **dadurch gekennzeichnet, dass** der Elektrodenträger (5) mit den Elektroden (3, 4) einerseits und die Steuerungseinrichtung (6) andererseits als separate Bauteile ausgebildet sind, die miteinander in Verbindung stehen, wobei die Signalmittel (7) mit der Steuerungseinrichtung (6) fest verbunden sind.

5. Vorrichtung nach einem der Ansprüche 1 bis 4, **dadurch gekennzeichnet, dass** die Signalelemente (7', 7", 7'") Leuchtdioden sind, wobei die Leuchtdioden vorzugsweise Licht unterschiedlicher Farbe emittieren.

6. Vorrichtung nach Anspruch 5, **dadurch gekennzeichnet, dass** drei Leuchtdioden (7', 7", 7'") vorhanden sind.

7. Vorrichtung nach Anspruch 6, **dadurch gekennzeichnet, dass** die drei Leuchtdioden grünes, gelbes und rotes Licht emittieren.
